# EUROPEAN PATENT SPECIFICATION

(11) **EP 2 281 194 B1**
(45) Date of publication and mention of the grant of the patent: **21.11.2012**
(21) Application number: 09733976.6
(22) Date of filing: 24.04.2009
(51) Int. Cl.: G01N 33/543, G01N 33/553, G01N 33/58, C07F 1/12, C07K 17/14

(54) **MOLECULAR ASSEMBLY COMPRISING GOLD AND A LINKER FOR DETECTION OF BIOCHEMICAL ENTITIES**
MOLEKULARE ANORDNUNG MIT GOLD UND EINEM LINKER ZUM NACHWEIS BIOCHEMISCHER VERBINDUNGEN
ENSEMBLE MOLÉCULAIRE COMPRENANT DE L'OR ET UN LIANT POUR LA DÉTECTION D'ENTITÉS BIOCHIMIQUES

(30) Priority: 24.04.2008 SE 0800939
(43) Date of publication of application: 09.02.2011
(73) Proprietor: Svanova Biotech Ab, 751 83 Uppsala (SE)
(72) Inventor: HAMASUR, Beston, S-171 62 Solna (SE)
(74) Representative: Widén, Björn
(86) International application number: PCT/SE2009/050429
(87) International publication number: WO 2009/131536

(56) References cited:
- WO-A1-99/20649
- WO-A2-02/01228
- WO-A2-03/008376
- DE-A1- 10 144 251
- JP-A- 2006 038 594
- RU-C1- 2 013 374
- US-A1- 2007 249 063
- CORNEJO AA ET AL: "Gold(III) complexes of amides of EDTA (L,L1) and PDTA (L2,L3) as model systems for gold(III)-peptide/protein interactions", INORGANICA CHIMICA ACTA, vol. 349, 2003, pages 91-96, XP002631212,
- FAN YIN: "A novel capacitive sensor based on human serum albumin-chelant complex as heavy metal ions chelating proteins", ANALYTICAL LETTERS, TAYLOR & FRANCIS INC, US, vol. 37, no. 7, 1 December 2004 (2004-12-01), pages 1269-1284, XP008125887, ISSN: 0003-2719, DOI: DOI:10.1081/AL-120035897
- DATABASE WPI Week 199506, Derwent Publications Ltd., London, GB; AN 1995-042282, XP003026031 & RU 2 013 374 C1 (AS USSR PLANTS MICROORGANISMS BIOCHEM) 30 May 1994

## Description

### Field of the Invention

The present invention relates to a molecular assembly comprising a ligand, a linker and a detectable entity, wherein the ligand is covalently bound to the linker, and the linker is a chelating agent forming a coordination complex with the detectable entity.

### Background to the Invention

Antibodies conjugated with fluorescent dyes were prepared 50 years ago for the first microscopy detection of antigens in tissues. In 1970 enzyme labels for the first time provided a permanent coloured stain with higher resolution and greater stability than fluorescent tags. Since the introduction of gold labels to microscopy in 1971, gold labels have become very important for detection and quantification of proteins and nucleic acids when used in techniques such as blotting, flowcytometry, hybridisation, and DNA fingerprint identification. Among metallic colloids, gold remains one of the most widely used reagents with a history dating back to Faraday in 1857. RU 201374 describes the synthesis of gold particles from gold atoms and their conjugation to Streptavidin.

Superior stability, sensitivity and reproducibility of manufacture makes gold suitable for use in a variety of applications. In the electron microscope the gold particles are visible without further treatment. In the light microscope, however, the gold particles are made visible through a short and simple silver enhancing procedure. For detection by eye, gold particles will also reveal immobilised protein on a solid phase such as a blotting membrane through the accumulated red colour of the gold sol. Silver enhancement of this gold precipitate also gives improved detection sensitivity.

With correct conjugation chemistry gold particles may be coupled to a wide variety of molecules including proteins (e.g. antibodies), polypeptides, carbohydrates, polymers, polysaccharides, enzymes and nucleic acids.

There are several advantages in choosing gold particles as markers for microscopic and non-microscopic identification of target molecules. The abundance of different particle sizes to choose from makes it possible to study antigens microscopically over a wide range of magnifications and provides the opportunity for multiple labelling of antigens on tissue sections. While fluorescent labels and enzyme based colour reactions may fade with time and light exposure, gold particles do not disappear - gold particles provide a permanent label. Unlike some enzyme based detection systems, gold probes are essentially non-toxic, safe and easy to use. The good stability of a well-made gold conjugate gives the product a long shelf life whether frozen or stored at 4° C.

For the staining of proteins immobilised onto membranes, gold probes have unsurpassed detection sensitivity and resolution. Moreover gold probes are inexpensive in their application and their high sensitivity often allows valuable primary antibodies to be diluted significantly more than what is possible with the use of other detection systems.

Gold clusters in the range of 1.4 nm, ready for covalent conjugation of ligands, with two different surface chemistries (Maleimido and sulfo -N-hydrosuccinimide surfaces) have previously been synthesised. The disadvantages associated with these gold clusters however are; 1) their tiny sizes demand silver enhancement in all kinds of applications, 2) the reactive molecules at their surface operate at pH higher than 8.0, this property hamper their use with biomolecules having low isoelectric points, 3) larger molecules (such as IgM) are unable to bind to the gold cluster due to steric hindrance. Moreover gold clusters are difficult to produce since the required reagent components must be synthesised and several ultrafiltration and purification steps are needed.

Early rapid tests used colored latex to form the visual signal. Latex was originally, and still is, the prime labeling method used in agglutination tests. This is because of its predisposition to agglutinate in the presence of binding components. For rapid tests, in which stability of the conjugate is critical in order to avoid false positive results, this predisposition to agglutinate can become a major problem.

In the late 1980s a very important use for gold conjugates emerged related to their incorporation into rapid test immunoassays. Rapid tests utilising gold-labelled antibody to visualise the presence of an analyte in a liquid sample on antibody coated membranes have evolved to be the test of choice in cases where manual, fast, and inexpensive, non-instrument test formats are required. Such tests can be formatted either as a dipsticks or as devices enclosed within plastic housings.

Test methods for the diagnosis of various diseases are constantly being improved. Currently, immunological methods are among the most sensitive methods used to detect the presence of antigens or antibodies in samples. In such assays, colloidal gold particles are passively coated with antibodies specific to the agent of interest and dried on a conjugate pad. If antigen is present in a test sample, an immune complex will form between the colloidal gold-labelled detector antibody and the antigen in the test sample. The immune complex then ascends chromatographically up a strip that has been layered with a capture antibody, or directly through an antibody-coated membrane. The presence of a visual red stripe, due to the gold particles, is indicative of a positive test. The same principle can also be adapted for detection of specific antibodies if the counterpart antigens or peptide-carrier conjugate are immobilised on the strip. For certain analytes (e.g. hormones, drug of abuse, pesticides and polysaccharides) that can not easily be detected in this system, a competition type lateral flow immuno-chromatography system can be used.

Although antibodies can passively be adsorbed to gold colloid such direct coating have often been shown unsuccessful due to the partial dissociation of the antibodies, and free antibodies compete with the antibody-gold conjugate resulting in low sensitivity. Moreover incorrect orientation of adsorbed antibodies associated with passive coating results in conjugate possessing poor immunological activity. Passive adsorption to gold colloids is restricted to positively charged ligands and also to ligands with a strong hydrophobic character. Small and neutrally charged ligands of biological importance however can not bind directly to the gold particles. The disadvantages described above limits the use of gold probes in assays, such as rapid tests.

Several methods have been described to enhance adsorption of biological ligands to gold sols. Introduction of sulfhydryl groups into the protein molecule via thiolation is the most used method. Modification of ligand in this way can increase their ability to bind to gold sols as evidenced by increased resistance to salt-induced agglomeration. However, such modification is accompanied with significant loss of activity.

Protein modifiers such as N-succinimidyl S-acetylthioacetate (SATA), 2 iminothiolane (2 IT) or N-Succinimidyl 3-[2-pyridyldithio]-propionamido (SPDP) have been used for thiolation of antibodies. The sulfhydryl groups of the modified antibody can then strongly bind to the gold sol through dative bonding.

Sulfhydryl groups can also be created in antibody molecule by fragmentation of the antibody with pepsin or papain. The Fab fragments thus produced contain sulfhydryl ready for conjugation to gold sols.

Gold sol modification with thiol-containing reagents has also been described. Chemicals such as SH-R wherein R is CHO, -NH2, -COOH, SH and OH provide groups on the sol available for the binding or linking antibodies, antigens, or other ligands to the gold sol. The exposed groups on the sol then bind to the desired ligands by the action of linker molecules such as 1-ethyl-3-(3-dimethylaminopropyl) carbodiimide hydrochloride (EDC).

Not all types of modification described above are suitable for all ligands. While thiolation of some antibody classes significantly improved their ability to bind gold colloid other antibody isotypes were inactivated upon this treatment whereas other antibodies lost significant immunologic reactivity after pepsin/papain fragmentation.

### Summary of the invention

One object of the present invention is to eliminate or minimize the disadvantages of the prior art, for example the disadvantages associated with passive adsorption. It is a further object of the present invention to facilitate binding of ligands that are not capable of direct adsorption to gold particles.

This object is met by the present invention, which relates to a molecular assembly comprising a ligand, a linker and a detectable entity, wherein the ligand is covalently bound to the linker, and the linker is a chelating agent forming a coordination complex with the detectable entity, characterized in that the detectable entity comprises gold particles, and in that the chelating agent has the chemical structure 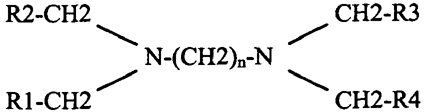 wherein R1, R2, R3, R4 can be the same or different, wherein R1-R4 is selected from the group consisting of -SH, -NH2, -COOH, hydrocarbons C1-C20 including or being substituted with at least one functional group comprising at least one heteroatom, and n ≥ 1, further characterized in that the ligand is selected from a group of chemical species including proteins, peptides, antibodies or fragments of antibodies, hormones, soluble antigens, carbohydrates, polysaccharides, DNA, RNA, and lipids, wherein the gold particles have divalent cations attached thereto to thereby exhibit reactive sites for forming a coordination complex with the chelating agent.

The present invention further relates to a process for producing the molecular assembly, wherein the process comprises the steps of:
i) mixing an aliquot of a solution comprising a ligand with an aliquot of a solution comprising an activated chelating agent, thereby binding the ligand covalently to the chelating agent, thereby forming a binder
ii) mixing an aliquot of gold particle suspension with an aliquot of a solution comprising divalent cations, to thereby exhibit reactive sites suitable for forming coordination complexes
iii) mixing an aliquot of the binder provided in step (i) with an aliquot of the gold particles produced in step (ii), thereby forming the molecular assembly in solution.

The present invention further relates to the use of a molecular assembly as a reagent in a method for detection of chemical and/or biochemical species.

The present invention further relates to a kit for detection of chemical and/or biochemical species, wherein the kit comprises a molecular assembly and a device; the device comprises a receptacle for receiving the molecular assembly, and a receptacle for receiving a sample comprising the species to be detected.

### Brief Description of the Invention

- Fig. 1: Synthesis of aminated EDTA
- Fig. 2: Synthesis of N-hydroxysuccinimide activated EDTA
- Fig.3: Synthesis of maleimide activated EDTA
- Fig. 4: Coupling of carbonyl containing ligands to aminated EDTA
- Fig.5: Coupling of thiol containing ligands to maleimide activated EDTA
- Fig.6: Coupling of amine containing ligands to NHS activated EDTA
- Fig.7: Scheme representing gold particles dressed with a ligand-EDTA conjugate

### Detailed Description of the Invention

The present invention proposes a rapid technique, easy to use for production of stable, sensitive and specific gold-ligand molecular assemblies to be used in a variety of applications. The invention takes advantage of the strong chelating capacity (i.e. the strong binding capacity) as well as the reactive group constituent of chelating agents.

Chelating agents are compounds capable of forming coordinate bonds with metals through two or more atoms of the organic compound. Such organic compounds are called chelating agents. The compound formed by a chelating agent and a metal is called a chelate, and the coordinate bonds formed are relatively strong.

The present invention relates to a molecular assembly comprising a ligand, a linker and a detectable entity, wherein the ligand is covalently bound to the linker, and the linker is a chelating agent forming a coordination complex with the detectable entity, characterized in that the detectable entity comprises gold particles, and in that the chelating agent has the chemical structure 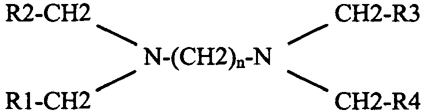 wherein R1, R2, R3, R4 can be the same or different, wherein R1-R4 is selected from the group consisting of -SH, -NH2, -COOH, hydrocarbons C1-C20 including or being substituted with at least one functional group comprising at least one heteroatom, and n ≥ 1.

In one embodiment of the present invention the gold particles are colloidal.

It is understood that a heteroatom is any atom different from carbon (C) and hydrogen (H).

In one embodiment of the present invention the heteroatoms are selected from a group of heteroatoms consisting of S, N, and O.

The ligand is covalently bound to the linker, and the binding of the ligand is typically to at least one of R1-R4.

According to the present invention, the linker can be any chelating agent capable of covalently binding the ligand in question.

Suitable chelating agents are: ethylenediaminetetraacetate (EDTA), diethylenetriamine-N,N,N',N',N"-pentaacetic acid (DTPA), and ethylene glycol bis (2-aminoethyl ether)-N,N,N'N'-tetraacetic acid (EGTA) trans-1,2-cyclohexylenediaminetetraacetic acid (DCTA), ethylenedinitrilotetrakis(methylphosphonic acid (EDTP), 1,4,7,10-tetraazacyclododecanetetraacetic acid (DOTA), N,N'-bis-(pyridoxal-5-phosphate)ethylenediamine-N,N'-diacet ic acid (DPDP), N-N'-bis(pyridoxal-5-phosphate)-trans-1,2-cyclohexyl-diamine -N,N'-diacetic acid (DPCP). The chelate can be mono-, bi-, tri-, tetra-, penta-, or hexadanate.

Only chelates with reactive groups capable of covalent binding to desired ligands can be used as a linker according to the present invention. Another important property of the chelate is the binding strength (pKa value) of the chelate with its ion. Not all kinds of ion are suitable with the gold colloid, therefore a correct chelate must be selected accordingly. The chelate must also tolerate transformation without losing ability to form a firm complex with the ion. For example, EDTA is a hexadentate chelating agent with four carboxyl groups. Occupation of these groups during conjugation to ligands affects the property of the EDTA, transforming it from hexadentate to bidentate but still with full capacity to form complex with the ions. These properties make EDTA an attractive chelate in this invention.

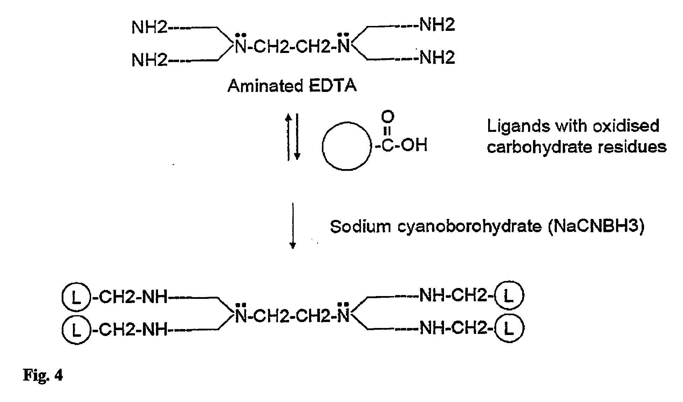

The choice of potentially useful conjugation chemistries is determined by the chemical nature of the ligand, and the analyte to be determined. The invented method is mild, stable and results in the formation of a stable molecular assembly which is essentially fully reactive, for example immunologically fully reactive. Advantages associated with the present invention, compared to previously known techniques, include:
- Improved sensitivity, as the molecular assembly according to the present invention enables binding of more analyte molecules per gold particle.
- Mild treatment of the ligands, no fragmentation step is necessary as, for example, for Fab fragment generation.
- Steric hindrance is minimized by the action of a spacer arm constituent of the chelate. This property allows immobilisation of larger antigens like IgM, which is not enabled by previously known conjugation methods.
- Suitability for all kinds of ligands irrespective of isoelectric point and/or isotypes (in the case of antibodies).
- Resistance to the extreme pH conditions found in many cell compartments (e.g. vacuoles and lysosomes).
- Ligands can be immobilised in a correct orientation (i.e. without affecting the ligand binding characteristics regarding specificity or affinity) on gold through reductive amination of aldehyde groups created on the antibody (example 2) with amine groups of the modified chelate molecules (fig.4).
- Small and uncharged molecules (such as oligosaccharides) can be immobilised on gold with fully retained activity.
- Gold particles of any shape and size, typically with diameters ranging from 5-250 nm, can be used for conjugation without restriction and loss of sensitivity and stability, for example due to agglomeration effects. The gold particles are preferably colloidal.

In one embodiment of the present invention, R1, R2, R3, and/or R4 is a heterocycle.

In a further embodiment of the present invention, the heterocycle is N-hydroxysuccinimide ester.

In a further embodiment of the present invention, the heterocycle is maleimide.

In one embodiment of the present invention, the ligand is selected from a group of chemical species including proteins, peptides, antibodies or fragments of antibodies, hormones, soluble antigens, carbohydrates, polysaccharides, DNA, RNA, and lipids.

According to the present invention, the gold particles have been treated with divalent cations to thereby exhibit reactive sites for forming a coordination complex with the chelating agent.

The present invention further relates to a process for producing a molecular assembly, characterized in that the process comprises the steps of:
i) mixing an aliquot of a solution comprising a ligand with an aliquot of a solution comprising an activated chelating agent, thereby binding the ligand covalently to the chelating agent, thereby forming a binder
ii) mixing an aliquot of gold particle suspension with an aliquot of a solution comprising divalent cations, to thereby form reactive sites suitable for forming coordination complexes
iii) mixing an aliquot of the binder provided in step (i) with an aliquot of the gold particles having cations attached thereto produced in step (ii) thereby forming the molecular assembly in solution.

It is understood that the term activated means: a molecule or reagent that is chemically modified to exhibit one or more reactive group(s) capable of reacting with another group to form a stable bond. A reaction of an aldhyde group (-CHO) with a -NH2 group is an example of activation. Another example of activation is a reaction between an electrophile and a nucleophile.

In one embodiment of the present invention, the process further comprises the step of blocking uncoated sites on the gold particles, to prevent agglomeration of the particles, through addition of a suitable compound.

In a further embodiment of the present invention, the compound used to block uncoated sites on the gold particles is bovine serum albumin, casein, polyvinyl pyrolidone, or polyvinyl alcohol.

The present invention further relates to the use of the molecular assembly as a reagent in a method for detection of chemical and/or biochemical species.

It is understood that the chemical species can be any of the species selected from a group of chemical species including proteins, peptides, antibodies or fragments of antibodies, hormones, soluble antigens, carbohydrates, polysaccharides, DNA, RNA, and lipids.

It is further understood that the biochemical species can be any of the species selected from a group of biochemical species including cells, bacteria, and virus particles.

The present invention further relates to a kit for detection of chemical and/or biochemical species, wherein the kit comprises the molecular assembly and a device; the device comprises a receptacle for receiving the molecular assembly, a receptacle for receiving a sample comprising the species to be detected, a migration zone, and a detection zone for detecting the interaction between the species and the molecular assembly.

The molecular assembly according to the present invention is applicable within a number of application areas.

In one embodiment of the present invention, the molecular assembly is used for detection of chemical and/or biochemical species in systems for chromatography.

In a further embodiment, the molecular assembly is used for detection of chemical and/or biochemical species in systems for lateral flow chromatography.

In a further embodiment, the molecular assembly is used for detection of small molecules (Hormones, lipopolysaccharides-LPS, small peptides, drugs) which are not capable of binding in a sandwich format.

In one embodiment of the present invention, the molecular assembly is used for detection of chemical and/or biochemical species by the use of electron microscopy.

In one embodiment of the present invention, the molecular assembly is used for detection of chemical and/or biochemical species by the use of immunoblotting.

In a further embodiment, the molecular assembly is used for detection in DNA microarrays.

In a further embodiment, the molecular assembly is used for detection in agglutination assays (using large gold particles).

In a further embodiment, the molecular assembly is used for affinity purification of specific proteins. Antibodies to the target protein are first conjugated to the gold and then mixed with the protein mixture/ cell extract. After short incubation the target protein bound to the gold conjugate (the molecular assembly according to the present invention) is separated by the use of centrifugation and subsequently the protein can be dissociated from the conjugate by lowering the pH followed by centrifugation.

In a further embodiment, the molecular assembly is used for antibody production; non-immunogenic molecules such as lipids and carbohydrates can be conjugated to the gold before immunisation. This strategy enhances immunogenicity of the molecules due to better presentation and recognition of the molecules by the cells of the immune system.

In a further embodiment, the molecular assembly is used as a vehicle for delivery of drugs and genes to target cells. The great stability of the molecular assembly makes it suitable as a vehicle for delivery of drugs and genes to target cells. Further, their resistance to harsh environments such as the acidic environment of some cell compartments makes the conjugate excellent for use in cell biology.

In a further embodiment, the molecular assembly is used in immunohistochemistry, for example for localisation of heavy metals in organs and detection of proteins in cells of a tissue section.

### Example 1. Activation of the chelating agent.

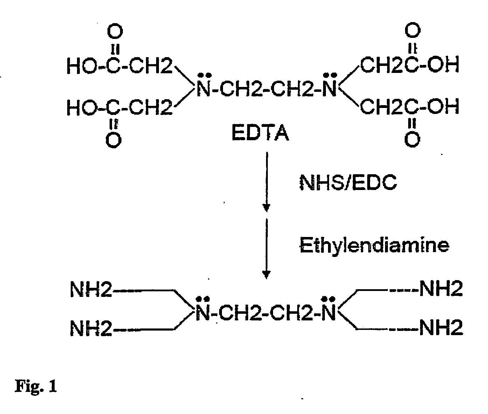

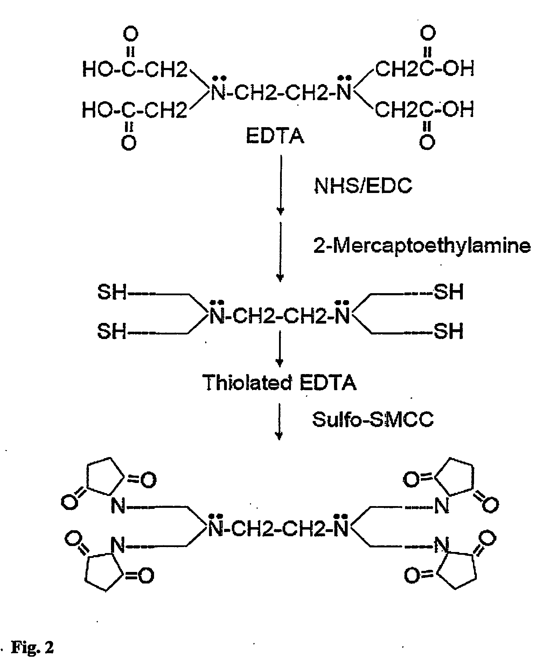

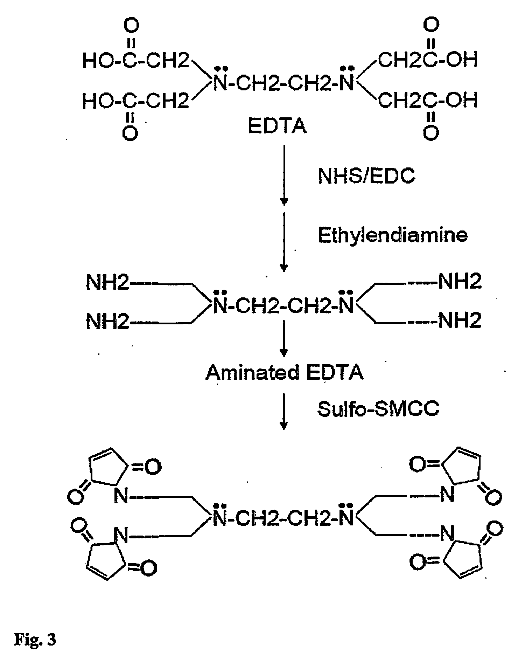

The carboxyl groups on the EDTA were first converted to amine groups (Fig.1) with ethylendiamine by the action of EDC and N-hydroxysuccinimide (NHS) or to thiol groups (Fig.2) with 2-mercaptoethylamine in the presence of EDC and NHS. After purification on metal chelating affinity column, the thiolated chelator then is activated with Sulfo-SMCC (Sulfosuccinimidyl 4-[*N*-maleimidomethyl] cyclohexane-1-carboxylate) and the resulting NHS-chelator purified by gel filtration on Biogel P-2 (Fig.2). In another experiment the amine groups of the modified EDTA were allowed to react with Sulfo-SMACC to form maleimide activated EDTA reactive to thiol containing ligands (Fig.3). Either reaction mixtures contain equimolar amounts of each reactant. This product is stable for years when kept frozen in aliquots.

### Example 2. Coupling of carbonyl containing ligands to aminated EDTA.

Ligands containing sugar moiety such as some receptors, hormones and antibodies and also LPS need to be activated first. In one experiment LPS treated with 0.3 molar sodium periodate in the dark for 15 minutes. This treatment results in creation of aldhyde groups in the sugar molecules and hence facilitates their conjugation to the amine- activated chelator through reductive amination (Fig.4). This product is stable for years when kept frozen in aliquots.

### Example 3. Coupling of thiol containing ligands to maleimide activated EDTA.

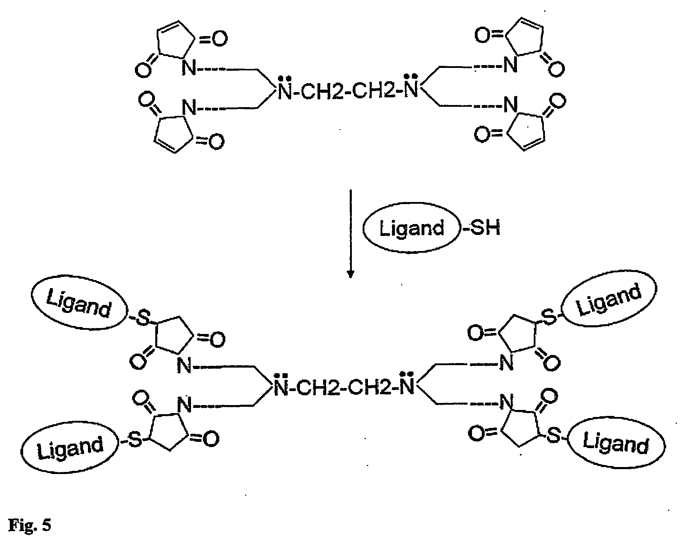

Ligands containing thiol groups can easily be coupled/conjugated to maleimide activated EDTA (Fig.5). This chemistry is suitable for Fab and (Fab')₂ fragments of antibodies and also proteins that contain cystine residue. Free thiol groups ready to react with maleimide-EDTA can easily be created from these ligands by the action of dithiothreitol (DTT) or any reducing agents. This product is stable for years when kept frozen in aliquots.

### Example 4. Coupling of amine containing ligands to NHS activated EDTA.

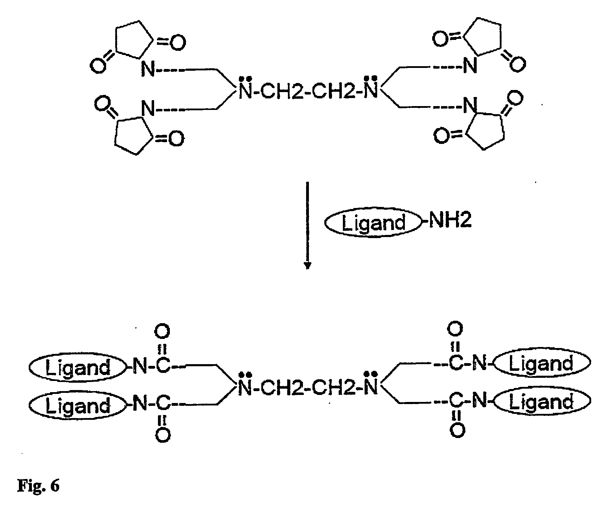

The reactive NHS ester created on the EDTA can readily react with the free amine groups in the ligands, especially with the lysine residues to form a stable covalent linkage (Fig.6). This product is stable for years when kept frozen in aliquots.

### Example 5. Treatment of gold particles with divalent or trivalent cations.

The net negative charges that are present on the gold particles can be used to accumulate suitable cations on the gold surface. Mn²⁺ was shown to be compatible with the gold. In one experiment when gold sols were incubated with radioactive positively charged amino acid lysine (¹⁴C-lysine) a substantial amount of radioactive material retained on the gold particles after extensive washing of the gold particles by centrifugation. However when gold particles were treated with manganese ions prior to the addition of ¹⁴C-lysine, no radioactivity could be measured after washing the gold indicating that the gold surface was preoccupied with cations. The optimal concentration of divalent cations required for this treatment, without destabilising the gold, must be determined by titration. In some experiments 1-5 mM of divalent cations were shown to be optimal.

### Example 6. Coupling of ligands to the activated chelating agent and their conjugation to gold.

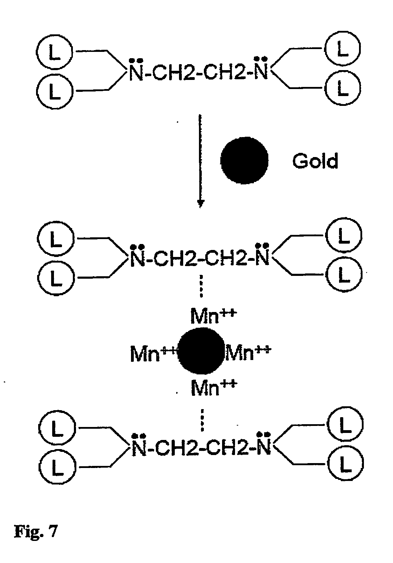

Dialyse a required amount of ligands overnight against proper buffer. For antibodies and peptides borate and phosphate buffer at optimal determined pH is recommended. For each millilitre of gold sols, premix approximately 20 mM of the activated chelator with approximately 30 micrograms of the ligands for 15 min with stirring before adding them to the gold sols (Fig. 4- Fig.6). In parallel treat the gold sol with optimal concentration of manganese as described above. Add the ligand-chelator complex to the treated gold sols very slow under vigorous stirring (Fig.7). After 15 min incubation, the coated gold sols were stabilized by blocking the uncoated sites on the gold sols through the addition of proper proteins or polymers such as; bovine serum albumin, casein, polyvinyl pyrolidone, polyvinyl alcohol, to prevent agglomeration. The reaction mixture was then centrifuged, and the gold conjugate (a loose precipitate) was collected from the bottom of the tube and further purified from excess ligand and aggregate by glycerol gradient centrifugation. Fractions that contain non-aggregated material as governed by electron microscopy were pooled and resuspended in a proper buffer. A proper buffer could be composed of a buffer like phosphate buffer saline, Tris saline, borate saline with additives such as sucrose, trehalose, glycerol, PVP,PVP and protein stabilizer like BSA, casein and various non-ionic and zwitterionic detergent and preservative like sodium azide, or merthiolate.

## Claims

1. Molecular assembly comprising a ligand, a linker and a detectable entity, wherein the ligand is covalently bound to the linker, and the linker is a chelating agent forming a coordination complex with the detectable entity, **characterized in that** the detectable entity comprises gold particles, and **in that** the chelating agent has the chemical structure 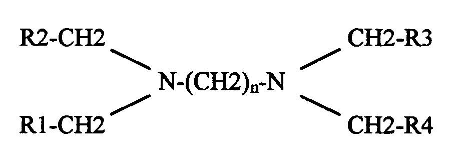 wherein R1, R2, R3, R4 can be the same or different, wherein R1-R4 is selected from the group consisting of -SH, -NH2, -COOH, hydrocarbons C1-C20 including or being substituted with at least one functional group comprising at least one heteroatom, and n ≥ 1, further **characterized in that** the ligand is selected from a group of chemical species including proteins, peptides, antibodies or fragments of antibodies, hormones, soluble antigens, carbohydrates, polysaccharides, DNA, RNA, and lipids wherein the gold particles have divalent cations attached thereto to thereby exhibit reactive sites for forming a coordination complex with the chelating agent.

2. Molecular assembly according to claim 1, **characterized in that** R1, R2, R3, and/or R4 is a heterocycle.

3. Molecular assembly according to claim 2, **characterized in that** the heterocycle is N-hydroxysuccinimide ester.

4. Molecular assembly according to claim 2, **characterized in that** the heterocycle is maleimide.

5. A process for producing a molecular assembly according to any of the claims 1-4, **characterized in that** the process comprises the steps of:
i) mixing an aliquot of a solution comprising a ligand with an aliquot of a solution comprising an activated chelating agent, thereby binding the ligand covalently to the chelating agent, thereby forming a binder
ii) mixing an aliquot of gold particle suspension with an aliquot of a solution comprising divalent cations, to thereby form reactive sites suitable for forming coordination complexes
iii) mixing an aliquot of the binder provided in step (i) with an aliquot of the gold particles having cations attached thereto produced in step (ii) thereby forming the molecular assembly in solution.

6. The process according to claim 5, **characterized in that** the process further comprises the step of blocking uncoated sites on the gold particles, to prevent agglomeration of the particles, through addition of a suitable compound.

7. The process according to claim 6, **characterized in that** the compound is bovine serum albumin, casein, polyvinyl pyrolidone, or polyvinyl alcohol.

8. Use of a molecular assembly according to any of the claims 1-4 as a reagent in a method for detection of chemical and/or biochemical species.

9. Use of a molecular assembly according to claim 8, wherein the chemical species is selected from a group of chemical species including proteins, peptides, antibodies or fragments of antibodies, hormones, soluble antigens, carbohydrates, polysaccharides, DNA, RNA, and lipids.

10. Use of a molecular assembly according to claim 8, wherein the biochemical species is selected from a group of biochemical species including cells, bacteria, and virus particles.

11. A kit for detection of chemical and/or biochemical species, wherein the kit comprises a molecular assembly according to any of the claims 1-4 and a device; the device comprises a receptacle for receiving the molecular assembly, a receptacle for receiving a sample comprising the species to be detected, a migration zone, and a detection zone for detecting the interaction between the species and the molecular assembly.

12. A kit for detection of chemical and/or biochemical species according to claim 11, wherein the chemical species is selected from a group of chemical species including proteins, peptides, antibodies or fragments of antibodies, hormones, soluble antigens, carbohydrates, polysaccharides, DNA, RNA, and lipids; and wherein the biochemical species is selected from a group of biochemical species including cells, bacteria, and virus particles.

## Patentansprüche

1. Molekulare Anordnung umfassend einen Liganden, einen Linker und eine nachweisbare Einheit, wobei der Ligand kovalent an den Linker gebunden ist und der Linker ein Chelatbildner ist, der einen Koordinationskomplex mit der nachweisbaren Einheit bildet, **dadurch gekennzeichnet, dass** die nachweisbare Einheit Goldpartikel umfasst und dass der Chelatbildner die chemische Struktur 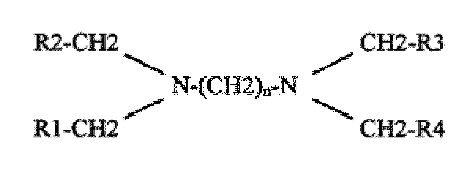 hat,
wobei R1, R2, R3, R4 gleich oder verschieden sein können, wobei R1-R4 ausgewählt sind aus der Gruppe bestehend aus -SH, -NH2, -COOH, Kohlenwasserstoffen C1-C120, die mindestens eine funktionelle Gruppe umfassen oder damit substituiert sind, die mindestens ein Heteroatom beinhaltet, und n ≥ 1, weiter **dadurch gekennzeichnet, dass** der Ligand ausgewählt ist aus einer Gruppe von chemischen Spezies einschließlich Proteinen, Peptiden, Antikörpern oder Antikörperfragmenten, Hormonen, löslichen Antigenen, Kohlehydraten, Polysacchariden, DNA, RNA und Lipiden, wobei die Goldpartikel zweiwertige Kationen haben, die daran gebunden sind, so dass sie reaktive Stellen aufweisen, um einen Koordinationskomplex mit dem Chelatbildner zu formen.

2. Molekulare Anordnung nach Anspruch 1, **dadurch gekennzeichnet, dass** R1, R2, R3 und/oder R4 ein Heterocyclus ist.

3. Molekulare Anordnung nach Anspruch 2, **dadurch gekennzeichnet, dass** der Heterocyclus N-Hydroxysuccinimidester ist.

4. Molekulare Anordnung nach Anspruch 2, **dadurch gekennzeichnet, dass** der Heterocyclus Maleimid ist.

5. Verfahren zur Herstellung einer molekularen Anordnung nach einem der Ansprüche 1 bis 4, **dadurch gekennzeichnet, dass** das Verfahren folgende Schritte umfasst:
i) Mischen eines Aliquots einer Lösung, die einen Liganden umfasst, mit einem Aliquot einer Lösung, die einen aktivierten Chelatbildner umfasst, so dass der Ligand kovalent an den Chelatbildner gebunden wird, wodurch ein Binder gebildet wird,
ii) Mischen eines Aliquots einer Goldpartikel-Suspension mit einem Aliquot einer Lösung, die zweiwertige Kationen umfasst, so dass reaktive Stellen gebildet werden, die geeignet sind, Koordinationskomplexe zu bilden,
iii) Mischen eines Aliquots des Binders, der in Schritt i) bereit gestellt wird, mit einem Aliquot der Goldpartikel, die Kationen daran gebunden haben, die in Schritt ii) hergestellt wurden, wodurch die molekulare Anordnung in Lösung gebildet wird.

6. Verfahren nach Anspruch 5, **dadurch gekennzeichnet, dass** das Verfahren weiter den Schritt der Blockierung von unbeschichteten Stellen auf den Goldpartikeln durch Zugabe einer geeigneten Verbindung umfasst, um das Agglomerieren der Partikel zu verhindern.

7. Verfahren nach Anspruch 6, **dadurch gekennzeichnet dass** die Verbindung Rinderserumalbumin, Casein, Polyvinylpyrrolidon oder Polyvinylalkohol ist.

8. Verwendung einer molekularen Anordnung nach einem der Ansprüche 1 bis 4 als ein Reagens in einem Verfahren zum Nachweis von chemischen und/oder biochemischen Spezies.

9. Verwendung einer molekularen Anordnung nach Anspruch 8, wobei die chemische Spezies ausgewählt ist aus einer Gruppe von chemischen Spezies einschließlich Proteinen, Peptiden, Antikörpern oder Antikörperfragmenten, Hormonen, löslichen Antigenen, Kohlehydraten, Polysacchariden, DNA, RNA und Lipiden.

10. Verwendung einer molekularen Anordnung nach Anspruch 8, wobei die biochemische Spezies ausgewählt ist aus einer Gruppe von biochemischen Spezies, einschließlich Zellen, Bakterien und Viruspartikeln.

11. Kit zum Nachweis von chemischen und/oder biochemischen Spezies, wobei der Kit eine molekulare Anordnung nach einem der Ansprüche 1 bis 4 und eine Vorrichtung umfasst; wobei die Vorrichtung umfasst: einen Behälter zur Aufnahme der molekularen Anordnung, einen Behälter zur Aufnahme einer Probe, die die Spezies, die nachgewiesen werden soll, umfasst, eine Migrationszone und eine Nachweiszone zum Nachweis der Interaktion zwischen der Spezies und der molekularen Anordnung.

12. Kit zum Nachweis von chemischen und/oder biochemischen Spezies nach Anspruch 11, wobei die chemische Spezies ausgewählt ist aus einer Gruppe von chemischen Spezies einschließlich Proteinen, Peptiden, Antikörpern oder Antikörperfragmenten, Hormonen, löslichen Antigenen, Kohlehydraten, Polysacchariden, DNA, RNA und Lipiden, und wobei die biochemische Spezies ausgewählt ist aus einer Gruppe von biochemischen Spezies einschließlich Zellen, Bakterien und Viruspartikeln.

## Revendications

1. Assemblage moléculaire comprenant un ligand, un lieur et une entité détectable, dans lequel le ligand est lié de manière covalente au lieur, et le lieur est un agent de chélation formant un complexe de coordination avec l'entité détectable, **caractérisé en ce que** l'entité détectable comprend des particules d'or, et **en ce que** l'agent de chélation a la structure chimique 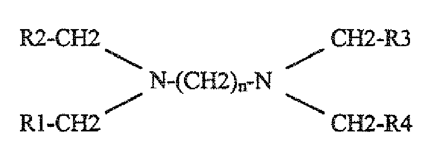 dans laquelle R1, R2, R3, R4 peuvent être identiques ou différents, R1-R4 étant choisis dans le groupe constitué par -SH, -NH2, -COOH, les hydrocarbures en C1-C20 contenant ou étant substitués par au moins un groupe fonctionnel comprenant au moins un hétéroatome, et n ≥ 1, **caractérisé, en outre, en ce que** le ligand est choisi dans un groupe d'espèces chimiques incluant les protéines, les peptides, les anticorps ou fragments d'anticorps, les hormones, les antigènes solubles, les glucides, les polysaccharides, l'ADN, l'ARN, et les lipides, dans lequel les particules d'or sont liées à des cations divalents pour présenter ainsi des sites réactifs pour former un complexe de coordination avec l'agent de chélation.

2. Assemblage moléculaire selon la revendication 1, **caractérisé en ce que** R1, R2, R3, et/ou R4 est (sont) un hétérocycle.

3. Assemblage moléculaire selon la revendication 2, **caractérisé en ce que** l'hétérocycle est un ester de N-hydroxysuccinimide.

4. Assemblage moléculaire selon la revendication 2, **caractérisé en ce que** l'hétérocycle est un maléimide.

5. Procédé de production d'un assemblage moléculaire selon l'une quelconque des revendications 1 à 4, **caractérisé en ce que** le procédé comprend les étapes consistant à :
i) mélanger une aliquote d'une solution comprenant un ligand avec une aliquote d'une solution comprenant un agent de chélation activé, pour lier ainsi le ligand de manière covalente à l'agent de chélation, et former ainsi un liant
ii) mélanger une aliquote d'une suspension de particules d'or avec une aliquote d'une solution comprenant des cations divalents, pour former ainsi des sites réactifs se prêtant à la formation de complexes de coordination
iii) mélanger une aliquote du liant obtenu à l'étape (i) avec une aliquote des particules d'or liées à des cations obtenues à l'étape (ii) pour former ainsi l'assemblage moléculaire en solution.

6. Procédé selon la revendication 5, **caractérisé en ce que** le procédé comprend, en outre, l'étape consistant à bloquer les sites non revêtus sur les particules d'or, pour empêcher l'agglomération des particules, par ajout d'un composé convenable.

7. Procédé selon la revendication 6, **caractérisé en ce que** le composé est la sérumalbumine bovine, la caséine, la polyvinylpyrrolidone, ou l'alcool polyvinylique.

8. Utilisation d'un assemblage moléculaire selon l'une quelconque des revendications 1 à 4 en tant que réactif dans un procédé de détection d'espèces chimiques et/ou biochimiques.

9. Utilisation d'un assemblage moléculaire selon la revendication 8, dans laquelle l'espèce chimique est choisie dans un groupe d'espèces chimiques comprenant les protéines, les peptides, les anticorps ou fragments d'anticorps, les hormones, les antigènes solubles, les glucides, les polysaccharides, l'ADN, l'ARN, et les lipides.

10. Utilisation d'un assemblage moléculaire selon la revendication 8, dans laquelle l'espèce biochimique est choisie dans un groupe d'espèces biochimiques comprenant les cellules, les bactéries, et les particules virales.

11. Trousse pour la détection d'espèces chimiques et/ou biochimiques, dans laquelle la trousse comprend un assemblage moléculaire selon l'une quelconque des revendications 1 à 4 et un dispositif, le dispositif comprenant un réceptacle pour recevoir l'assemblage moléculaire, un réceptacle pour recevoir un échantillon comprenant l'espèce à détecter, une zone de migration, et une zone de détection pour détecter l'interaction entre l'espèce et l'assemblage moléculaire.

12. Trousse pour la détection d'espèces chimiques et/ou biochimiques selon la revendication 11, dans laquelle l'espèce chimique est choisie dans un groupe d'espèces chimiques comprenant les protéines, les peptides, les anticorps ou fragments d'anticorps, les hormones, les antigènes solubles, les glucides, les polysaccharides, l'ADN, l'ARN, et les lipides, et dans laquelle l'espèce biochimique est choisie dans un groupe d'espèces biochimiques comprenant les cellules, les bactéries, et les particules virales.
